# EUROPEAN PATENT APPLICATION

(11) **EP 4 809 931 A1**
(43) Date of publication of application: **23.09.2026**
(21) Application number: 26162557.8
(22) Date of filing: 05.03.2026
(51) Int. Cl.: A61B 8/00, G01S 7/52

(54) **ULTRASOUND DIAGNOSTIC APPARATUS AND IMAGE GENERATION METHOD**

(30) Priority: 18.03.2025 JP 2025043975
(71) Applicant: FUJIFILM Corporation, Tokyo 106-8620 (JP)
(72) Inventor: KASAHARA, Eiji, Tokyo (JP)
(74) Representative: Parker, Andrew James

(57) **Abstract**

To support specifying a position of a tissue of interest in a living body.

In a past ultrasound examination, a processor generates a reference image including a body mark image and a probe mark, and also registers position information indicating a position of a tissue of interest in a living body. In a current ultrasound examination, the processor generates a second reference image that includes a body mark, a probe mark, and a pointer. The processor determines a display position of the pointer with respect to the probe mark based on the registered position information.

## Description

### BACKGROUND OF THE INVENTION

### 1. Field of the Invention

The present disclosure relates to an ultrasound diagnostic apparatus and an image generation method, and more particularly to a technique for generating images that support ultrasound examinations.

### 2. Description of the Related Art

An ultrasound diagnostic apparatus is used in ultrasound examinations of living bodies (subjects). The ultrasound diagnostic apparatus comprises an ultrasound probe (hereinafter simply referred to as a probe). In general, the transmitting and receiving surface of a probe is brought into contact with the surface of a living body, and in this state, ultrasonic waves are radiated from the probe into the living body, and the reflected waves from within the living body are detected by the probe.

In the ultrasound diagnostic apparatus, an ultrasound image is displayed on the screen of a display. A typical ultrasound image is a B-mode tomographic image (hereinafter simply referred to as a tomographic image). Usually, a body mark and a probe mark are displayed on the screen of the display together with the tomographic image. The body mark is a schematic diagram that represents the form of a specific portion of a living body or an image corresponding to the schematic diagram. The probe mark is a schematic diagram that represents a probe. An image including the body mark and the probe mark can be referred to as a reference image that represents the examination site, the probe contact position, or the cross-section position. For example, in the frozen state, the position and the rotation angle of the probe mark on the body mark are manually adjusted such that the position and the orientation of the probe at the time of acquiring the tomographic image are reproduced on the screen. Then, the display image, including the tomographic image and the reference image, is stored.

JP2005-118142A and JP2005-124712A disclose a 3D body mark and a 3D probe mark. JP2008-154833A discloses a 3D body mark and a scanning plane mark. JP2012-91042A discloses embedding an image in an examination report.

### SUMMARY OF THE INVENTION

Ultrasound examinations are periodically performed on the same subject for the purpose of follow-up observation or the like of a tissue of interest (for example, a lesion area). In such a case, in the current ultrasound examination, it is necessary to set the beam scanning plane at the same position as the position of the beam scanning plane (observation cross section) set in the past (usually the most recent) ultrasound examination. Furthermore, in the current ultrasound examination, it is necessary to specify the tissue of interest (image of the tissue of interest) within a tomographic image representing the beam scanning plane. In the current ultrasound examination, in a case in which the body mark and the probe mark stored in the past ultrasound examination are displayed, it is possible to support setting the beam scanning plane in the current ultrasound examination, that is, to support probe operation. However, it is not possible to specify the position of the tissue of interest in the living body from the displayed body mark and probe mark.

An object of the present disclosure is to support an examiner in an ultrasound examination. Alternatively, an object of the present disclosure is to make it possible to easily specify a tissue of interest within a tomographic image.

According to an aspect of the present disclosure, there is provided an ultrasound diagnostic apparatus comprising: a processor configured to generate a tomographic image based on data received from an ultrasound probe in contact with a living body, in which the processor is configured to: generate, in a past ultrasound examination, a first reference image including a first body image representing the living body and a first probe image representing the ultrasound probe, the first reference image being displayed together with the tomographic image, and register position information indicating a position of a tissue of interest in the living body; generate, in a current ultrasound examination, a second reference image including a second body image corresponding to the first body image, a second probe image corresponding to the first probe image, and a pointer representing the position of the tissue of interest, the second reference image being displayed together with the tomographic image; and determine, in a case of generating the second reference image, a display position of the pointer with respect to the second probe image based on the position information.

According to another aspect of the present disclosure, there is provided an image generation method executed in an ultrasound diagnostic apparatus, the method comprising: generating a tomographic image based on data received from an ultrasound probe in contact with a living body; generating, in a past ultrasound examination, a first reference image including a first body image representing the living body and a first probe image representing the ultrasound probe, the first reference image being displayed together with the tomographic image, and registering position information indicating a position of a tissue of interest in the living body; generating, in a current ultrasound examination, a second reference image including a second body image corresponding to the first body image, a second probe image corresponding to the first probe image, and a pointer representing the position of the tissue of interest, the second reference image being displayed together with the tomographic image; and determining, in a case of generating the second reference image, a display position of the pointer with respect to the second probe image based on the position information.

According to the aspect of the present disclosure, it is possible to support an examiner in an ultrasound examination. Alternatively, according to the aspect of the present disclosure, it is possible to easily specify a tissue of interest within a tomographic image.

### BRIEF DESCRIPTION OF THE DRAWINGS

FIG. 1 is a block diagram showing a configuration example of an ultrasound diagnostic apparatus according to an embodiment.
FIG. 2 is a diagram showing an example of a management table.
FIG. 3 is a diagram showing a first reference image displayed in a past ultrasound examination.
FIG. 4 is a diagram showing a relationship between a scanning range and a line segment.
FIG. 5 is a diagram showing a second reference image displayed in a current ultrasound examination.
FIG. 6 is a flowchart showing an operation of the ultrasound diagnostic apparatus in a past ultrasound examination.
FIG. 7 is a flowchart showing an operation of the ultrasound diagnostic apparatus in a current ultrasound examination.
FIG. 8 is a diagram showing a modification example of the second reference image.

### DESCRIPTION OF THE PREFERRED EMBODIMENTS

Hereinafter, an embodiment will be described with reference to the drawings.

### (1) Overview of Embodiment

An ultrasound diagnostic apparatus according to an embodiment includes a processor that generates a tomographic image based on data received from an ultrasound probe in contact with a living body. The processor generates, in a past ultrasound examination, a first reference image including a first body image representing a living body and a first probe image representing an ultrasound probe, the first reference image being displayed together with the tomographic image, and also registers position information indicating a position of a tissue of interest in the living body. The processor generates, in a current ultrasound examination, a second reference image including a second body image corresponding to the first body image, a second probe image corresponding to the first probe image, and a pointer representing the position of the tissue of interest, the second reference image being displayed together with the tomographic image. In a case of generating the second reference image, the processor determines the display position of the pointer with respect to the second probe image based on the position information.

In general, ultrasound examinations are repeatedly performed on a specific living body (a specific subject) for the purpose of follow-up observation or the like. Tissues of interest that were observed in a past (usually most recent) ultrasound examination are also observed in the current ultrasound examination. According to the above configuration, position information indicating the position of the tissue of interest in the past ultrasound examination is registered. In the current ultrasound examination, the display position of the pointer with respect to the second probe image is determined based on the registered position information. Therefore, by observing the second reference image displayed on the display, the examiner can recognize the approximate position of the tissue of interest in the living body, particularly by specifying the display position of the second probe image on the second body image and the display position of the pointer with respect to the second probe. The examiner operates the probe such that a tomographic image similar to that displayed in the past ultrasound examination is displayed in the current ultrasound examination.

The processor may be configured by one or more devices. The first body image and the second body image each include a body mark to be described later. The first probe image and the second probe image each include a probe mark to be described later. The pointer is a display element displayed on the second probe image, and is configured by, for example, a mark, a symbol, a figure, or the like. In the embodiment, the aspect of the second body image is the same as the aspect of the first body image. The aspect of the second probe image is the same as the aspect of the first probe image. As long as the lengths of the two line segments in the two probe images are the same, the colors or shapes of the two probe images may be different. The position information is generated based on user designation or based on the results of automatic detection of a tissue of interest. In the embodiment, a pointer is displayed on the second probe image. However, the pointer may be displayed near the second probe image, for example, to be in contact with the second probe image or to be close to the second probe image with a gap therebetween.

In the embodiment, the position of the tissue of interest includes a scanning position within the electronic scanning range. The second probe image has a line segment. One end of a line segment corresponds to one end of the electronic scanning range, and the other end of the line segment corresponds to the other end of the electronic scanning range. The processor determines, in the current ultrasound examination, the display position of the pointer between one end and the other end of the line segment. The configuration associates the line segment of the second probe image with the electronic scanning range. The maximum electronic scanning range may be associated with the line segment, or the actually set electronic scanning range may be associated with the line segment.

In the embodiment, the first ratio is a ratio related to an electronic scanning range, specifically, a ratio of the portion from one end to the scanning position to the entire range from one end to the other end. The second ratio is a ratio related to a line segment, specifically, a ratio of the portion from one end to the display position to the entire length from one end to the other end. The second ratio is equal to the first ratio. According to this configuration, by referring to the position of the pointer with respect to the line segment (second ratio), the scanning position where the tissue of interest exists within the electronic scanning range can be easily specified. The electronic scanning range is a linear range or an arc-shaped range.

In the embodiment, the processor registers, in a past ultrasound examination, information indicating the position and the rotation angle of the first probe image on the first body image. The processor determines, in the current ultrasound examination, the position and the rotation angle of the second probe image on the second body image based on information indicating the position and the rotation angle of the first probe image on the first body image. In a case of registering the position information, information indicating the position and the rotation angle of the first probe image may be registered. The information indicating the position and the rotation angle of the first probe image is, for example, PM information, which will be described later. The position information may be registered at the time of storing an image. The position and the rotation angle of the first probe image on the first body image are designated by a user or detected by a positioning system that detects the position and the orientation of the ultrasound probe. The rotation angle of the probe image is, for example, a rotation angle around the center point of the probe image.

In the embodiment, the first reference image includes a first pointer representing the position of the tissue of interest. The processor determines, in the past ultrasound examination, the display position of the first pointer on the first probe image based on position information. The pointer included in the second reference image is a second pointer corresponding to the first pointer. The display position of the second pointer on the second probe image is the same as the display position of the first pointer on the first probe image. According to this configuration, the second reference image similar to the first reference image is displayed in the current ultrasound examination. It may be understood that the first reference image is reproduced.

In the embodiment, the second reference image includes a third probe image representing the ultrasound probe in contact with the living body in the current ultrasound examination. The third probe image represents the current position and rotation angle of the ultrasound probe. For example, the current position and rotation angle of the ultrasound probe may be specified based on information from a positioning system.

In the embodiment, the processor registers, in a past ultrasound examination, the position information based on representative coordinates, which are coordinates designated by a user on a tomographic image or coordinates detected in a tomographic image. For example, the user designates the representative coordinates of the tissue of interest. Alternatively, the representative coordinates of a tissue of interest are detected by an object detector.

In the embodiment, the position information is information indicating the scanning position corresponding to the representative coordinates, or information indicating the display position on the first probe image. The information indicating a scanning position is, for example, a beam address. The information indicating a display position is, for example, a numerical value according to a scale defined on the first probe image.

The image generation method according to an aspect of the present disclosure is executed in an ultrasound diagnostic apparatus and includes first to fourth steps. In the first step, a tomographic image is generated based on data received from an ultrasound probe in contact with a living body. In the second step, a first reference image is generated in a past ultrasound examination, the first reference image including a first body image representing a living body and a first probe image representing an ultrasound probe, the first reference image being displayed together with the tomographic image. In the second step, position information indicating a position of a tissue of interest in the living body is registered. In the third step, a second reference image is generated in a current ultrasound examination, the second reference image including a second body image corresponding to the first body image, a second probe image corresponding to the first probe image, and a pointer representing the position of the tissue of interest, the second reference image being displayed together with the tomographic image. In the fourth step, in a case of generating the second reference image, a display position of the pointer with respect to the second probe image is determined based on the position information.

A program for executing the image generation method is installed in a storage unit within the ultrasound diagnostic apparatus via a network or via a portable storage medium. Such a program may be referred to as a program product. The storage unit in the ultrasound diagnostic apparatus is generally a non-transitory storage medium.

### (2) Details of Embodiment

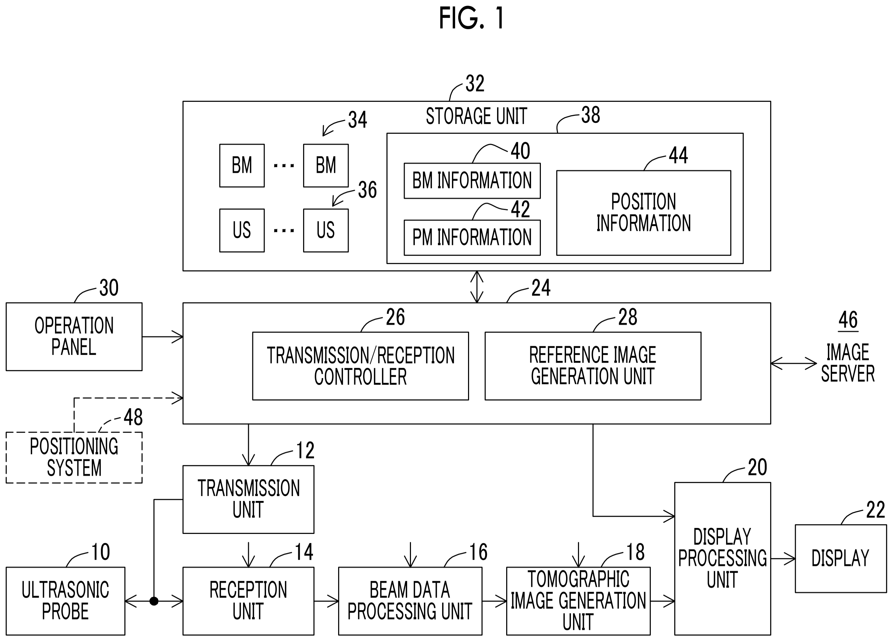

FIG. 1 is a block diagram showing a configuration of an ultrasound diagnostic apparatus according to an embodiment. An ultrasound diagnostic apparatus is a medical apparatus used to execute ultrasound examinations in medical institutions such as hospitals. In the embodiment, the part to be subjected to ultrasound diagnosis is, for example, the abdomen.

An ultrasound probe 10 is a portable transceiver that is held and operated by an examiner (such as a doctor or a laboratory technician). The transmitting and receiving surface of the ultrasound probe 10 (specifically, the surface of an acoustic lens) is brought into contact with the surface of the abdomen of the subject. While observing tomographic images displayed in real time, the ultrasound probe 10 is manually scanned along the surface of the abdomen.

In the shown configuration example, the ultrasound probe 10 comprises a transducer array consisting of a plurality of transducers arranged one-dimensionally. An ultrasound beam (a transmission beam) is formed by the transducer array. The ultrasound beam is electronically scanned to form a beam scanning plane within the subject. The beam scanning plane is the observation plane, that is, the two-dimensional data acquisition region. As ultrasound beam electronic scanning methods, an electronic sector scanning method and an electronic linear scanning method are known. A convex scanning of the ultrasound beam may be performed. A 2D transducer array may be provided in the ultrasound probe 10, and volume data may be acquired from within the living body by two-dimensional scanning with an ultrasound beam.

A transmission unit 12 is an electronic circuit that functions as a transmission beam former. Specifically, during transmission, the transmission unit 12 supplies a plurality of transmission signals in parallel to the transducer array. This forms a transmission beam. During reception, in a case in which a reflected wave from within the living body reaches the transducer array, a plurality of received signals are output in parallel from the plurality of transducers. A reception unit 14 is an electronic circuit that functions as a reception beam former. In the reception unit 14, a plurality of received signals are phased and added (delayed and added), thereby generating beam data (received beam data).

Incidentally, for each electronic scanning, a plurality of pieces of beam data arranged in the electronic scanning direction are generated, and these constitute received frame data corresponding to the beam scanning plane. Each piece of beam data is composed of a plurality of pieces of echo data aligned in the depth direction.

The received frame data sequence output from the reception unit 14 is sent to a tomographic image generation unit 18 via a beam data processing unit 16. The beam data processing unit 16 has an envelope detector, a logarithmic converter, and the like. The beam data processing unit 16 processes the individual beam data that constitute each piece of received frame data.

The tomographic image generation unit 18 is configured by a processor that generates a display frame data sequence based on the received frame data sequence. Specifically, the tomographic image generation unit 18 has a digital scan converter (DSC). A DSC has a coordinate conversion function, a pixel interpolation function, a frame rate conversion function, and the like. The display frame data sequence corresponds to a tomographic image sequence, and a moving image is formed by the tomographic image sequence. The display frame data sequence is sent to the display processing unit 20.

The display processing unit 20 has an image combining function, a color calculation function, and the like. In the display processing unit 20, a display image to be displayed on a display 22 is generated. Specifically, a display image is generated by combining the tomographic image with a reference image, which will be described in detail below.

The beam data processing unit 16, the tomographic image generation unit 18, and the display processing unit 20 may be configured by a CPU, which will be described later. The display 22 is composed of an organic EL display, a liquid crystal display, or the like.

Specifically, a processor 24 is configured by a CPU that executes a program. In the embodiment, the processor 24 functions as a transmission/reception controller 26 and a reference image generation unit 28. The transmission/reception controller 26 controls the formation of transmission beams and reception beams. Specifically, the transmission/reception controller 26 controls the operations of the transmission unit 12 and the reception unit 14.

The reference image generation unit 28 generates a reference image to be displayed together with the tomographic image. As will be described in detail later, the reference image has a body mark as a body image, a probe mark as a probe image, and a pointer representing a position of a tissue of interest. In the following, in some cases, a reference image displayed in a past ultrasound examination of a specific subject will be referred to as a first reference image, and a reference image displayed in a current ultrasound examination of a specific subject will be referred to as a second reference image.

An operation panel 30 is connected to the processor 24. The operation panel 30 has a trackball, a plurality of switches, a plurality of knobs, a keyboard, and the like. An image server 46 is connected to the processor 24 via a network. Further, a storage unit 32 is connected to the processor 24. The storage unit 32 is configured by, for example, one or more semiconductor memories.

The storage unit 32 stores a plurality of tomographic images 36 stored by the examiner's store operation. The plurality of tomographic images 36 may be transferred to the image server 46. In the storage unit 32, a plurality of body marks 34 are stored as a plurality of graphic images. A specific body mark to be actually displayed is selected from among the plurality of body marks 34 by a user or is automatically selected. For example, a specific body mark is automatically selected with reference to preset information.

The storage unit 32 includes a management table 38. The management table 38 has a plurality of records corresponding to a plurality of ultrasound examinations. One record is registered usually for one ultrasound examination. However, a plurality of records may be registered for one ultrasound examination. Each record has body mark (BM) information 40, probe mark (PM) information 42, and position information 44. The body mark information 40 is information indicating a body mark type. The probe mark information 42 is information indicating the position and the rotation angle of the probe mark on the body mark. The body mark information 40 may include information indicating the body mark type. The position information is information indicating the position of the tissue of interest in the living body. The position information will be described in detail later.

A positioning system 48 may be provided to measure the position and the orientation of the ultrasound probe 10. Generally, the positioning system 48 is composed of a magnetic sensor, a magnetic field generator, and a positioning controller. The magnetic sensor is provided in the ultrasound probe 10. The magnetic sensor detects a magnetic field generated by the magnetic field generator. Accordingly, three-dimensional coordinate information of the magnetic sensor is obtained. Based on the three-dimensional coordinate information, the position and the orientation of the ultrasound probe 10 are specified. The position information from the positioning controller is output. Other positioning systems may also be provided.

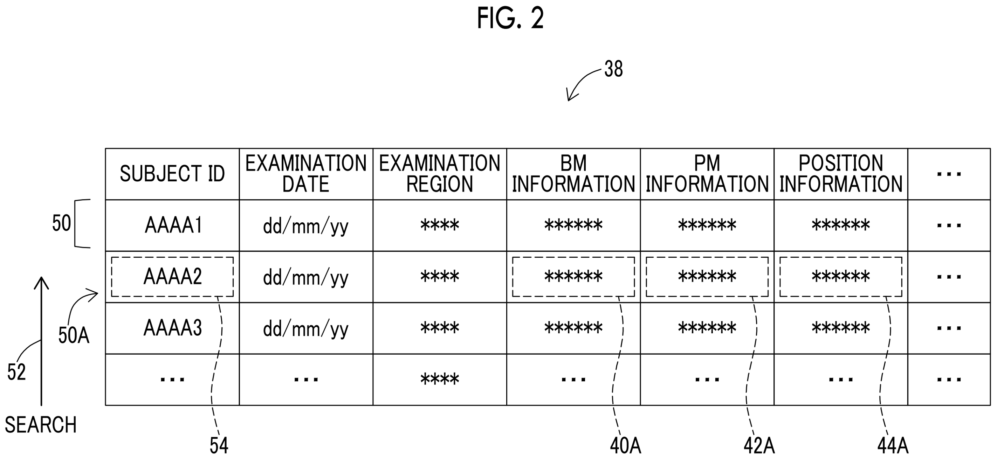

In FIG. 2, an example of the management table 38 is shown. The management table 38 has a plurality of records 50 corresponding to the plurality of ultrasound examinations. Each record 50 includes subject ID information, information specifying the examination date, information specifying the examination region (diagnosis region), BM information, PM information, position information, and the like.

At the start of the current ultrasound examination, for example, the subject ID is used as a search key to search for the most recent record (the record corresponding to the most recent ultrasound examination) that includes the same subject ID as the search key (see reference numeral 52). In the shown example, a record 50A having a subject ID 54 is specified. Prior to generating a second reference image, BM information 40A, PM information 42A, and position information 44A included in the record 50A are referenced. In searching, a combination of the examiner ID and the examination region may be used as a search key.

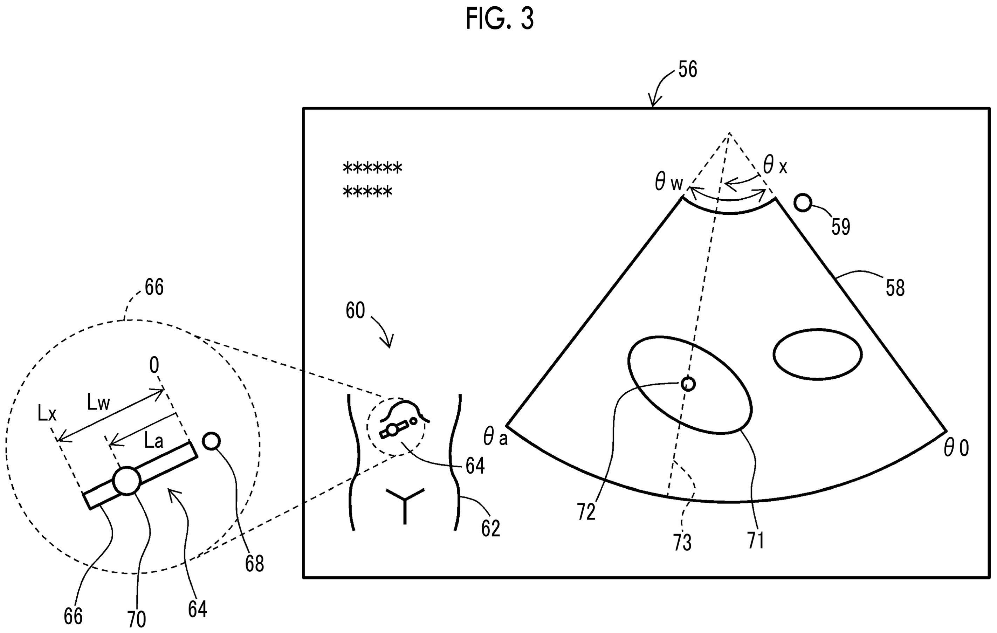

FIG. 3 shows an image 56 displayed in a past ultrasound examination. The image 56 includes a tomographic image 58 and a first reference image 60. The tomographic image 58 includes a tissue of interest (an image of the tissue of interest) 71. An imaginary line 73 is a line that originates from the origin and passes through representative coordinates 72 of the tissue of interest 71. The representative coordinates 72 are, for example, coordinates designated by a user or coordinates specified by a lesion area detector. The representative coordinates 72 are, for example, the center coordinates or centroid coordinates of the tissue of interest 71.

A mark 59 indicates a starting end of the electronic scan. The electronic scanning range is represented by θw. The scanning position corresponding to the line 73 is represented by θx. The scanning position θx is a beam address corresponding to the representative coordinates 72 within the electronic scanning range.

The first reference image 60 includes a body mark (first body mark) 62, a probe mark (first probe mark) 64, and a pointer (first pointer) 70. Reference numeral 66 denotes an enlarged view of the probe mark 64. The probe mark 64 includes a line segment 66 and a mark 68. The line segment represents the probe main body, the beam scanning plane, or the upper side of the beam scanning plane. The mark 68 indicates the starting end of the electronic scan. The pointer 70 is displayed on the line segment 66. The pointer 70 is a mark that indicates the position of the tissue of interest 71 in the living body. The pointer 70 is displayed in a superimposed manner on the line segment 66 between one end and the other end.

That is, the line segment 66 corresponds to the electronic scanning range θw, and a display position La of the pointer 70 corresponds to the scanning position θx. In FIG. 3, in a case in which the position of the tissue of interest 71 shifts to the right, the pointer 70 shifts to the right on the line segment 66. On the other hand, in a case in which the position of the tissue of interest 71 shifts to the left, the pointer 70 shifts to the left on the line segment 66.

In a case in which the representative coordinates 72 are designated by the user, the display position of the pointer 70 is determined after the representative coordinates 72 are designated. Before the representative coordinates 72 are designated, the display position of the pointer 70 may be set to the intermediate position of the line segment 66, or the pointer 70 may be hidden.

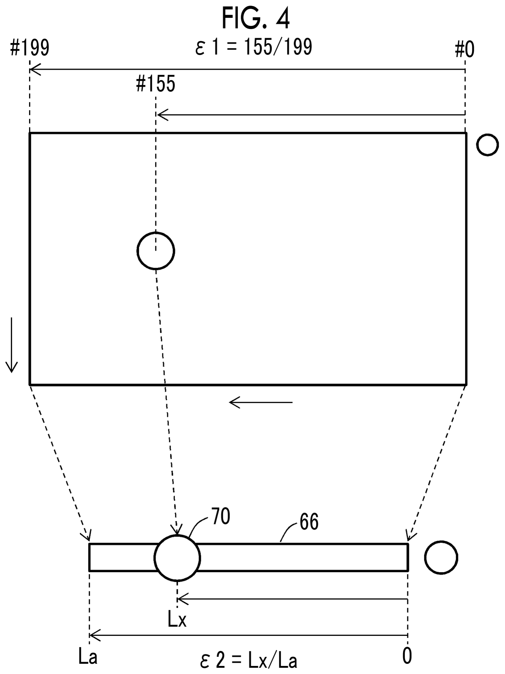

FIG. 4 shows a relationship between the electronic scanning range and the line segment. In FIG. 4, the upper part shows the beam scanning plane, and the lower part shows the probe mark and the first pointer. The first beam address in the beam scanning plane is indicated by #0, and the last beam address is indicated by #199. The beam address corresponding to representative coordinates of the tissue of interest is indicated by #155. In the beam scanning range, there are 199 beam addresses from one end to the other end. There are 155 beam addresses from one end to the beam address #155. In such a case, a first ratio ε1 is 155/199.

The probe mark has the line segment 66. The line segment has an entire length from 0 to La. A second ratio ε2 is calculated as Lx/La. Here, Lx indicates the length from one end of the line segment 66 to the display position of the pointer. Lx is calculated so that ε2 is equal to ε1. That is, the display position of the pointer is calculated.

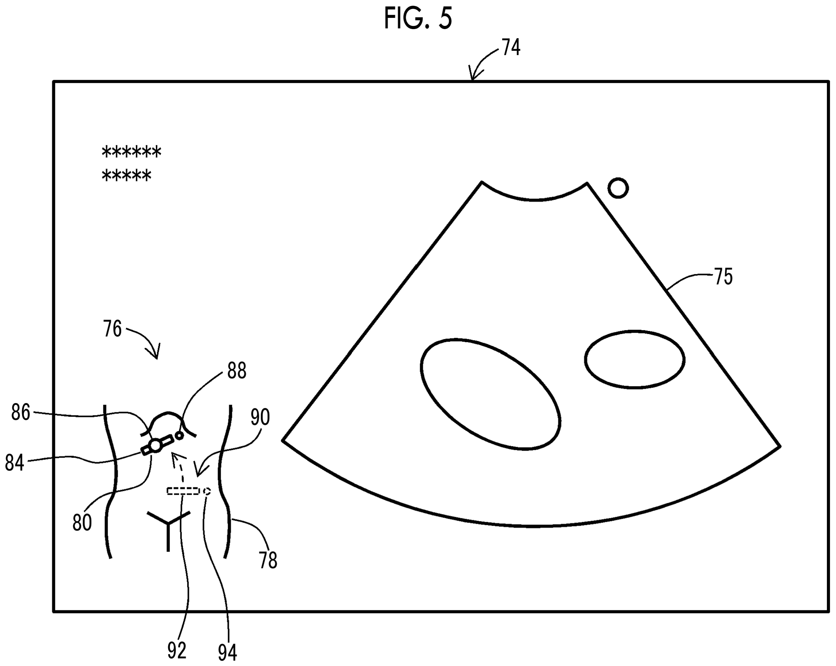

FIG. 5 shows an image 74 displayed in a current ultrasound examination. The image 74 includes a tomographic image 75 and a second reference image 76. The second reference image 76 is basically the same image as the first reference image displayed in the past ultrasound examination. It may be understood that the first reference image is reproduced in the current ultrasound examination.

The second reference image 76 includes a body mark (second body mark) 78, a probe mark (second probe mark) 80, and a pointer (second pointer) 86. The second body mark 78 corresponds to the first body mark. The second probe mark 80 corresponds to the first probe mark. The second pointer 86 corresponds to the first pointer.

In a case of generating and displaying the first reference image, the processor registers body mark information and probe mark information in the management table, and also registers position information of the tissue of interest. The probe mark information is information indicating the position and the rotation angle of the probe mark on the body mark. The position information of the tissue of interest corresponds to a beam address specified by a line passing through the representative coordinate of the tissue of interest.

In a case of generating and displaying the second reference image 76, the processor specifies the body mark type based on the body mark information, and specifies the position and the rotation angle of the probe mark 80 on the body mark 78 based on the probe mark information. The probe mark 80 is composed of a line segment 84 and a mark 88. The processor determines the display position of the pointer 86 on the line segment 84 in the probe mark 80 based on the position information.

With reference to the second reference image 76, the examiner can obtain probe operation information for displaying a tomographic image corresponding to the tomographic image displayed in the past ultrasound examination.

As a part of the second reference image 76, a probe mark (third probe mark) 90 may be displayed, which indicates the current position and rotation angle of the probe. The probe mark 90 is composed of a line segment 92 and a mark 94. For example, the position and the rotation angle of the probe mark 90 may be determined based on information output from a positioning system. In such a case, after the examiner operates the probe, the probe mark 90 overlaps the probe mark 80. A display mode of the probe mark 90 may be different from a display mode of the probe mark 80.

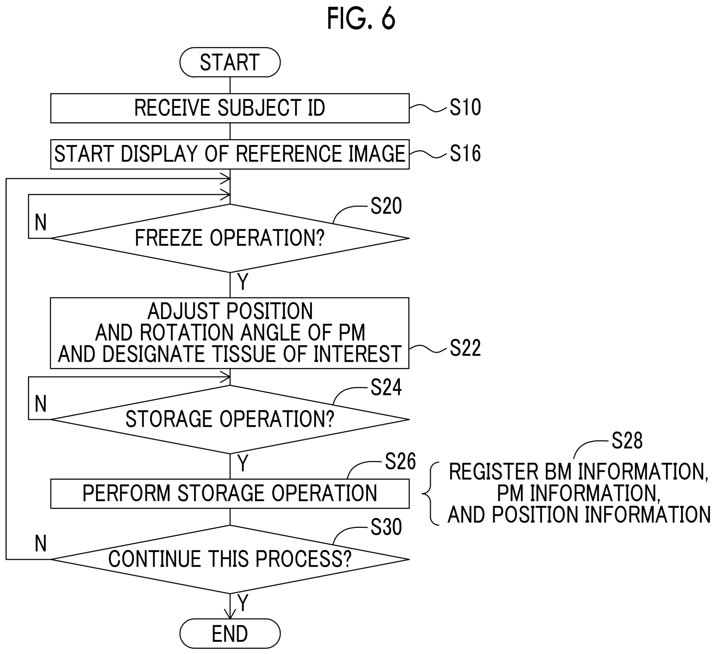

FIG. 6 is a flowchart showing an operation of the ultrasound diagnostic apparatus in a past ultrasound examination.

In S10, the subject ID is received. In S16, the display of the reference image starts. Further, in S16, transmission and reception are started. In a case in which a tissue of interest such as a lesion area appears on a tomographic image, the examiner performs a freeze operation. In S20, a freeze operation is received. Accordingly, transmission and reception are stopped, and the tomographic image is displayed as a still image.

In S22, the position and the rotation angle of the probe mark are adjusted by the examiner. In such a case, the position and the rotation angle of the probe mark are changed based on the position and the orientation of the probe at the point in time of acquiring the tomographic image to be stored. In addition, in S22, the examiner designates the representative coordinates of the tissue of interest. Accordingly, the pointer is displayed or the display position of the pointer is changed. Specifically, the display position of the pointer is determined so that the second ratio specified by the display position of the pointer is the same as the first ratio specified by the representative coordinates.

In S24, a storage operation is received. Accordingly, the display image including the tomographic image and the first reference image is stored (see S26). At this time, the BM information, the PM information, and the position information are registered in the management table (see S28). In S30, it is determined whether to continue this process. In a case in which the process is continued, transmission and reception are resumed.

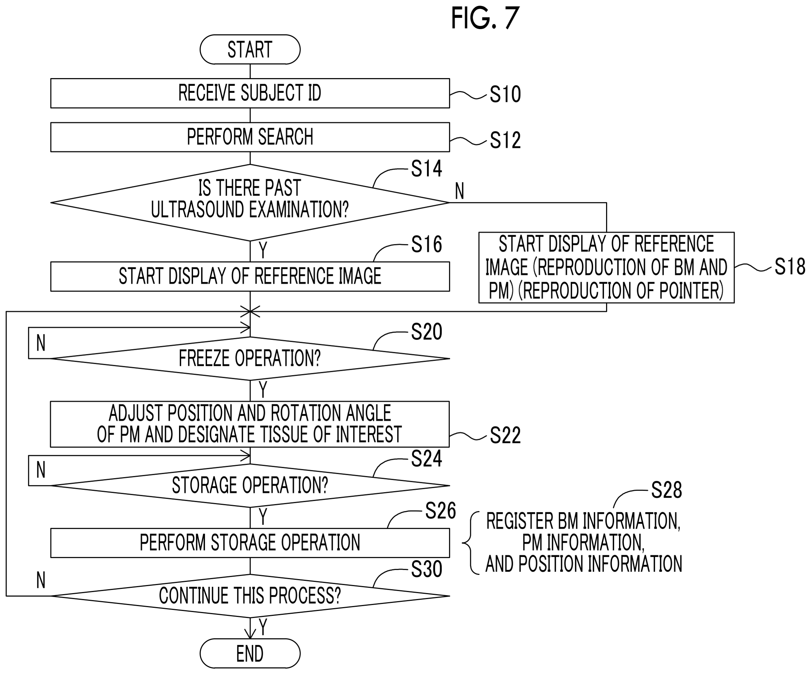

FIG. 7 is a flowchart showing an operation of the ultrasound diagnostic apparatus in a current ultrasound examination. In FIG. 7, the same steps as those shown in FIG. 6 are denoted by the same reference numerals, and the description thereof is omitted.

In S12, a management table is searched using the subject ID as a search key. That is, a search is made as to whether or not there is a past ultrasound examination that corresponds to the current ultrasound subject. In a case in which it is determined in S14 that there is no past ultrasound examination, display of the reference image (which may also be called the first reference image) starts in S18.

In a case in which it is determined in S14 that there is a past ultrasound examination, in S16, a second reference image is generated based on the BM information, the PM information, and the position information read from the management table, and the display of the second reference image starts.

More specifically, a body mark is generated based on the BM information, a probe mark is generated based on the PM information, and further a pointer is generated based on the position information. The second reference image is the same as the first reference image, and thus it may be understood that the first reference image is reproduced. As a part of the second reference image, the third probe mark described above may be generated and displayed.

In a case in which the second reference image is displayed, the execution of S22 may be omitted. Alternatively, in S22, correction of the position and the rotation angle of the probe mark and re-designation of the tissue of interest may be received. Alternatively, designation of the position and the rotation angle of the third probe mark may be received. In S28, the current BM information, the current PM information, and the current position information are registered in the management table.

As described above, with the image generation method according to the embodiment, in the current ultrasound examination, the examiner can recognize the target position and the target rotation angle of the probe through observation of the second reference image.

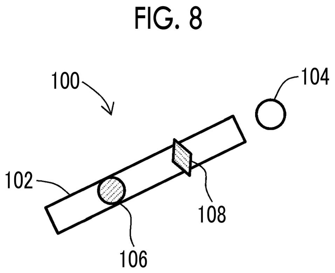

In FIG. 8, a modification example of the second reference image is shown. In the second reference image, the probe mark 100 includes a line segment 102 and a mark 104. A pointer 106 indicating the position of a first tissue of interest and a pointer 108 indicating the position of a second tissue of interest are displayed in a superimposed manner on the line segment 102. In this way, the positions of a plurality of tissues of interest may be displayed as a plurality of pointers. In such a case, it is desirable to make the shapes of the plurality of pointers different from each other.

In the present embodiment, each process is executed by any computer. In addition, any computer may execute these types of processing by a processor as hardware, a program as software, or a combination thereof. In such a case, the processor is configured to execute various types of processing in the present embodiment in cooperation with the program, and may function as each unit or each means in the present embodiment. In addition, the execution order of the processing by the processor is not limited to the above-described order and may be changed as appropriate. Any computer may be a general-purpose computer, a computer for specific use, a workstation, or another system capable of executing each processing.

The processor may be composed of one or a plurality of pieces of hardware, and types of hardware are not limited. For example, the processor may be configured with hardware such as a central processing unit (CPU), a micro processing unit (MPU), a programmable logic device such as a field-programmable gate array (FPGA), a dedicated circuit for executing specific processing such as an application-specific integrated circuit (ASIC), a graphic processing unit (GPU), or a neural processing unit (NPU). Additionally, the types of hardware may be a combination of different types of hardware. In a case in which the plurality of types of hardware are configured to execute one or a plurality of types of processing of a certain processor, the plurality of types of hardware may exist in devices physically separated from each other or may exist in the same device. Furthermore, in any of the embodiments, the order of each processing performed by the processor is not limited to the above-described order, and may be changed as appropriate. The hardware is composed of an electrical circuit (circuitry) in which circuit elements such as semiconductor elements are combined.

The program may be software such as firmware or a microcode. Furthermore, the program may be, for example, a program module group, and each function thereof may be implemented by a processor configured to execute each function. The program may be a program code or a plurality of code segments stored in one or a plurality of non-transitory computer-readable media (for example, a storage medium and other storages). The program may be stored in the plurality of non-transitory computer-readable media existing in physically separated devices. The program code or the code segment may represent any combination of procedures, functions, subprograms, routines, subroutines, modules, software packages, classes, instructions, data structures, or program statements. The program code or the code segments may be connected to other code segments or hardware circuits by transmitting and receiving information, data, an argument, a parameter, or content of a memory.

### Explanation of References

10: ultrasound probe
18: tomographic image generation unit
20: display processing unit
28: reference image generation unit
38: management table

## Claims

1. An ultrasound diagnostic apparatus comprising:
a processor configured to generate a tomographic image based on data received from an ultrasound probe in contact with a living body,
wherein the processor is configured to:
generate, in a past ultrasound examination, a first reference image including a first body image representing the living body and a first probe image representing the ultrasound probe, the first reference image being displayed together with the tomographic image, and register position information indicating a position of a tissue of interest in the living body;
generate, in a current ultrasound examination, a second reference image including a second body image corresponding to the first body image, a second probe image corresponding to the first probe image, and a pointer representing the position of the tissue of interest, the second reference image being displayed together with the tomographic image; and
determine, in a case of generating the second reference image, a display position of the pointer with respect to the second probe image based on the position information.

2. The ultrasound diagnostic apparatus according to claim 1,
wherein the position of the tissue of interest includes a scanning position within an electronic scanning range,
the second probe image has a line segment,
one end of the line segment corresponds to one end of the electronic scanning range,
another end of the line segment corresponds to another end of the electronic scanning range, and
the processor is configured to determine, in the current ultrasound examination, the display position of the pointer between the one end and the other end of the line segment.

3. The ultrasound diagnostic apparatus according to either claim 1 or claim 2,
wherein, in a case in which a first ratio is defined as a ratio of a portion of the electronic scanning range from the one end to the scanning position with respect to an entire range from the one end to the other end, and a second ratio is defined as a ratio of a portion of the line segment from the one end to the display position with respect to an entire length from the one end to the other end,
the second ratio is equal to the first ratio.

4. The ultrasound diagnostic apparatus according to any one of claims 1 to 3,
wherein the processor is configured to:
register, in the past ultrasound examination, information indicating a position and a rotation angle of the first probe image on the first body image; and
determine, in the current ultrasound examination, a position and a rotation angle of the second probe image on the second body image based on the information indicating the position and the rotation angle of the first probe image on the first body image.

5. The ultrasound diagnostic apparatus according to any one of claims 1 to 4,
wherein the first reference image includes a first pointer representing the position of the tissue of interest,
the processor is configured to determine, in the past ultrasound examination, a display position of the first pointer with respect to the first probe image based on the position information,
the pointer included in the second reference image is a second pointer corresponding to the first pointer, and
a display position of the second pointer with respect to the second probe image is the same as the display position of the first pointer with respect to the first probe image.

6. The ultrasound diagnostic apparatus according to any one of claims 1 to 5,
wherein the second reference image includes a third probe image representing the ultrasound probe in contact with the living body in the current ultrasound examination.

7. The ultrasound diagnostic apparatus according to any one of claims 1 to 6,
wherein the processor is configured to register, in the past ultrasound examination, the position information based on representative coordinates, which are coordinates designated by a user on the tomographic image or coordinates detected within the tomographic image.

8. The ultrasound diagnostic apparatus according to any one of claims 1 to 7,
wherein the position information is information indicating a scanning position corresponding to the representative coordinates, or information indicating the display position with respect to the first probe image.

9. An image generation method in an ultrasound diagnostic apparatus, the method comprising:
generating a tomographic image based on data received from an ultrasound probe in contact with a living body;
generating, in a past ultrasound examination, a first reference image including a first body image representing the living body and a first probe image representing the ultrasound probe, the first reference image being displayed together with the tomographic image, and registering position information indicating a position of a tissue of interest in the living body;
generating, in a current ultrasound examination, a second reference image including a second body image corresponding to the first body image, a second probe image corresponding to the first probe image, and a pointer representing the position of the tissue of interest, the second reference image being displayed together with the tomographic image; and
determining, in a case of generating the second reference image, a display position of the pointer with respect to the second probe image based on the position information.

10. A program executed in an ultrasound diagnostic apparatus, the program causing the ultrasound diagnostic apparatus to implement:
generating a tomographic image based on data received from an ultrasound probe in contact with a living body;
generating, in a past ultrasound examination, a first reference image including a first body image representing the living body and a first probe image representing the ultrasound probe, the first reference image being displayed together with the tomographic image, and registering position information indicating a position of a tissue of interest in the living body;
generating, in a current ultrasound examination, a second reference image including a second body image corresponding to the first body image, a second probe image corresponding to the first probe image, and a pointer representing the position of the tissue of interest, the second reference image being displayed together with the tomographic image; and
determining, in a case of generating the second reference image, a display position of the pointer with respect to the second probe image based on the position information.
